# NEW EUROPEAN PATENT SPECIFICATION

(11) **EP 0 969 846 B2**
(45) Date of publication and mention of the opposition decision: **25.08.2010**
(45) Mention of the grant of the patent: 07.01.2004
(21) Application number: 98916948.7
(22) Date of filing: 13.03.1998
(51) Int. Cl.: A61K 31/557, A61K 9/00, A61P 27/06

(54) **COMPOSITIONS AND METHODS FOR REDUCING OCULAR HYPERTENSION**
ZUSAMMENSETZUNGEN UND VERFAHREN ZUR BEHANDLUNG DES ERHÖHTEN AUGENINDRUCKES
COMPOSITIONS ET PROCEDES POUR REDUIRE L'HYPERTENSION OCULAIRE

(30) Priority: 17.03.1997 US 819221
(43) Date of publication of application: 12.01.2000
(73) Proprietor: Novartis AG, 4056 Basel (CH); Novartis Pharma GmbH, 1230 Wien (AT)
(72) Inventor: REED, Kenneth, Warren, Lawrenceville, GA 30243 (US); YEN, Shau-Fong, Atlanta, GA 30319 (US); SOU, Mary, Alpharetta, GA 30202 (US); PEACOCK, Regina, Flinn, Alpharetta, GA 30005 (US)
(74) Representative: de Weerd, Petrus G.W.
(86) International application number: PCT/EP1998/001483
(87) International publication number: WO 1998/041208

(56) References cited:
- EP-A- 0 458 587
- WO-A-92/13836
- WO-A-95/30420
- WO-A1-97//29752
- US-A- 5 558 876

## Description

The invention relates broadly to ophthalmic technology. More specifically, this invention relates to therapeutic treatment of the eye or ocular tissue to reduce elevated intraocular pressure, for example, elevated intraocular pressure which is associated with glaucoma.

The use of prostaglandins as medicaments is known in the art. For example, U.S. Patent Nos. 5,106,869 and 5,221,763 disclose the use of 13,14-dihydro-15-keto PGFs (F-series prostaglandins) for raising blood pressure without substantial ephemeral depression of blood pressure which occurs with many PGFs.

Also, the use of prostaglandin active agents to treat certain ocular conditions is known in the art. The term "prostaglandin active agent", as used herein, refers to prostaglandins, salts thereof, and mixtures thereof. For example, U.S. Patent Nos. 5,001, 153; 5,151,444; 5,166,178 and 5,212,200 disclose the use of 13,14-dihydro-15-keto prostaglandin metabolites to reduce ocular pressure without causing a transient ocular hypotension response which prostaglandins usually cause. These patents discuss a collyrium (i.e., eyewash) which may include water as a diluent, BAK or NaCl as an isotonizing agent, borate or phosphate buffers, EDTA as a stabilizer, and a polysorbate surfactant.

Furthermore, U.S. Patent No. 5,208,256, issued to Ryuji Ueno on May 4, 1993 teaches a method of treating ocular hypotension by ocularly administering a combination of 13,14-dihydro-15-keto-20-loweralkylprostaglandin, or salt or ester thereof, and a polyoxyethylene-sorbitan unsaturated higher aliphatic acid monoester. Preferred examples of the latter includes miristoleic acid, palmitoleic acid, oleic acid, gadoleic acid and linoleic acid. Polyoxyethylene (20) sorbitan monooleate is also known as Polysorbate 80 and sold, *inter alia,* under the names SORLATE, CRILLET, TWEEN 80, MONITAN and OLOTHORB.

With regard to ophthalmic surfactants, CREMOPHOR has been used as a surfactant in eye drops (See Japanese Patent 07316060, filed on Dec. 16, 1994). CREMOPHOR is a ethoxylated, hydrogenated caster oil, which is also referred to as a polyoxyethylene hardened caster oil. However, the use of CREMOPHOR with prostaglandins in an ophthamic delivery system has not been disclosed or suggested.

WO 95 30420 A describes an ophthalmic composition comprising a prostaglandin as drug, benzalkonium chloride as ophthalmic preservative, a non-ionic tonicity adjusting agent, an ophthalmic carrier, and vitamin E tocopherol polyethylene glycol succinate for solubilizing the drug and reducing the discomfort and irritation associated with the topical ophthalmic administration of the drug.

US-A-5,558,876 describes a similar topical ophthalmic composition comprising a prostaglandin as drug, benzalkonium chloride as ophthalmic preservative, a non-ionic tonicity adjusting agent, an ophthalmic carrier, vitamin E tocopherol polyethylene glycol succinate, and caffeine for increasing the preservative-efficacy of benzalkonium chloride.

EP-A-0 458 587 describes a composition for ocular administration and useful the treatment of ocular hypertension comprising a synergistic combination of a prostaglandin and a sympathomimetic agent and optionally other usual ingredients including carriers, ionic tonicity agents and ophthalmic preservatives.

WO 92 13836 discloses ophthalmic compositions comprising a prostaglandin active agent, an ophthalmic preservative, a tonicity adjusting agent, and ophthalmic carrier and, optionally, a suitable amount of a surfactant, preferably a polyoxyethylene sorbitan fatty acid ester. The tonicity adjustors are not limited according to the teaching of said reference.

While prostaglandin active agents, especially 13,14-dihydro-15-keto prostaglandin metabolites, are advantageous in reducing intraocular pressure, there is a need to improve the efficacy of these medicaments. In addition, there is a need for improvements in the preservative effectiveness of ophthalmic prostaglandin compositions which include surfactants, while maintaining good efficacy and good ocular tolerance. Furthermore, the improvements in shelf life of ophthalmic prostaglandin compositions are desirable. Also, it is always desirable to reduce the manufacturing difficulties. Moreover, there is a need for a prostaglandin-containing ophthalmic composition which can be manufactured with a minimum of complexities and which exhibits a balance of efficacy, preservative effectiveness, ocular tolerance, and a long shelf life.

An object of the invention is to improve the efficacy of 13,14-dihydro-15-keto-20-ethyl-PGF₂α isopropyl ester-containing ophthalmic compositions.

Another object of the invention is to improve the preservative effectiveness of 13,14-dihydro-15-keto-20-ethyl-PGF₂α isopropyl ester-containing ophthalmic compositions.

Still another object of the invention is to improve shelf life of 13,14-dihydro-15-keto-20-ethyl-PGF₂α isopropyl ester-containing ophthalmic compositions.

Yet another object of the invention is to reduce the complexity of manufacturing a 13,14-dihydro-15-keto-20-ethyl-PGF₂α isopropyl ester-containing ophthalmic composition.

A further object of the invention is to produce a 13,14-dihydro-15-keto-20-ethyl-PGF₂α isopropyl ester-containing ophthalmic composition with a desirable balance of efficacy, preservative effectiveness, ocular tolerance, and shelf life.

These and other objects and advantages of the invention are achieved with the various embodiments of the present prostaglandin-containing ophthalmic compositions, methods of use and methods of manufacture.

The instant invention therefore relates to a composition, comprising:
(a) a prostaglandin active agent which is a 13,14-dihydro-15-keto-20-ethyl-PGF₂α isopropyl ester, salts thereof, and mixtures thereof;
(b) an ophthalmic preservative;
(c) a non-ionic tonicity adjusting agent, wherein said non-ionic tonicity adjusting agent is present in a concentration sufficient to:
   (i) adjust the tonicity of the composition and
   (ii) increase the preservative effectiveness;
(d) an ophthalmically acceptable carrier, and
further comprising 0.1 to 5 weight percent of a non-ionic surfactant selected from the group consisting of polyoxyethylene sorbitan fatty acid esters, polyoxyethylene alkyl ethers, and mixtures thereof.

The various embodiments of the invention offer a number of improvements in prostaglandin compositions which are useful, *inter alia,* for reducing intraocular pressure. The compositions are especially useful in treating elevated intraocular pressure associated with glaucoma. Accordingly, all of the components of the compositions are preferably ophthalmically acceptable, at the concentrations of use and under the conditions in which they are applied. An "ophthalmically acceptable" component, as used herein, refers to a component which will not cause any appreciable ocular damage or ocular discomfort at the intended concentration and over the time of intended use.

The invention embraces several embodiments, some of which are outlined below-to improve the reader's understanding. One group of embodiments of the invention are ophthalmic compositions which are useful in reducing intraocular pressure, especially intraocular pressure which is associated with glaucoma. The ophthalmic compositions include an amount of a prostaglandin active agent selected from 13,14-dihydro-15-keto-20-ethyl-PGF₂α isopropyl ester, salts thereof, or combinations thereof, which is effective in treating elevated intraocular pressures However, a person having ordinary skill in the art may vary some of the elements of the embodiments without departing from the spirit and scope of the invention.

One embodiment of the invention is a composition which has a reduced concentration of strong preservative, and correspondingly, generates less ocular irritation. Unexpectedly, it has been found that the use of certain non-ionic tonicity adjusting agents enhances the preservative effectiveness of strong preservatives in compositions containing 13,14-dihydro-15-keto-20-ethyl-PGF₂α isopropyl ester. This allows for a reduced concentration of strong preservatives in the composition. In addition, chelating agents may be added to further boost preservative efficacy and reduce the required concentration of strong preservative.

In particular, the complete erradication of *Pseudomonas Aeriginosa* is desired. While benzalkonium chloride (BAK) kills nearly all *Pseudomonas,* there may remain some which are resistant to BAK. Over time, the BAK-resistant *Pseudomonas* may propagate to a concentration which is unacceptable. Thus, it is preferably to include a preservative efficacy enhancer to eliminate BAK-resistant *Pseudomonas.*

It is preferable that the preservative efficacy enhancer or second preservative be a well tolerated component which acts via a mechanism which differs from BAK. The strong preservative (e.g., BAK) will handle the bulk of the bioburden. The use of the second well tolerated preservative or enhancer insures complete kill of contaminating microbes and yet minimizes ophthalmic irritation as compared to using abnormally high concentrations of BAK. This is accomplished by choosing a well tolerated additive whose mechanism of action differs from the strong preservative.

A preferred class of preservative efficacy enhancers are chelating agents, such as calcium chelating agents. A preferred calcium chelating agent is ethylene diamine tetraacetate (EDTA). EDTA has been shown to assist in the erradication of BAK-resistant *Pseudomonas* without substantially altering ophthalmic compatibility or prostaglandin efficacy. In addition, EDTA offers the advantage of simultaneously acting as a buffer.

Thus, in a preferred embodiment, the composition includes a chelating agent (e.g., edetate sodium). These compositions are especially advantageous in that preservative effectiveness is improved relative to a composition containing a strong preservative alone. This allows for a reduction in the required concentration of the strong preservative, and accordingly less ophthalmic irritation.

Another embodiment of the invention is a composition containing 13,14-dihydro-15-keto-20-ethyl-PGF₂α isopropyl ester which has an advantageously reduced total surfactant concentration. It is generally desirable to minimize the concentration additives to an ophthalmic formulation in order to minimize potential ocular irritation associated with the additives. However, in order to solubilize, 13,14-dihydro-15-keto-20-ethyl-PGF₂α isopropyl ester a surfactant is typically required. It has been unexpectedly discovered that the combination of two or more non-ionic surfactants, as opposed to a single surfactant, can reduce the total concentration of surfactant required to achieve a given level of solubility of 13,14-dihydro-15-keto-20-ethyl-PGF₂α isopropyl ester. Thus, this embodiment of the invention relates to a composition which includes (1) 13,14-dihydro-15-keto-20-ethyl-PGF₂α isopropyl ester, (2) a first non-ionic surfactant (e.g., Polysorbate 80), (3) a second non-ionic surfactant [e.g., a BRIJ surfactant] and (4) an ophthalmically acceptable carrier. This embodiment of the invention offers advantages in reduced ocular irritation and reduced raw material (surfactant) requirements.

Yet another embodiment of the invention relates to the difficulties in achieving solubility of 13,14-dihydro-15-keto-20-ethyl-PGF₂α isopropyl ester. In order to solubilize the active agent, a non-ionic surfactant, preferably Polysorbate 80, is added to the formulation. Thus, increasing the 13,14-dihydro-15-keto-20-ethyl-PGF₂α isopropyl ester concentration to the preferred ranges described herein requires a corresponding increase in the surfactant concentration, in order to maintain the prostaglandin in solution. However, the Polysorbate 80 surfactant deactivates the commonly used ophthalmic preservative benzalkonium chloride (BAK). Thus, an increase in surfactant reduces the preservative effectiveness. In sum, an increase in therapeutic efficacy which is achieved by increasing active agent concentration results in the need for an increase in Polysorbate 80 concentration and therefore a decrease in preservative effectiveness. Accordingly, improvements in both preservative effectiveness and efficacy of the cited formulations are difficult to achieve.

However, it has been unexpectedly found that the use of non-ionic tonicity adjusting agents appreciably improves the action of the preservative in the presence of surfactant. Thus, in order to minimize the aforementioned preservative deactivation problem, a preferred composition is a composition wherein said preservative (b) is a strong preservative (e.g., BAK) and said non-ionic surfactant (e) increases the solubility of 13,14-dihydro-15-keto-20-ethyl-PGF₂α isopropyl ester but decreases the preservative effectiveness of the strong preservative (e.g., Polysorbate 80); which composition further comprises (g) a preservative enhancer which increases the effectiveness of the strong preservative (e.g., mannitol or EDTA). Thus, the efficacy and preservative effectiveness may be simultaneously improved in the present formulations, while maintaining a solution form, by optimizing the concentrations of active agent, surfactant, non-ionic tonicity adjusting agent, and preservative.

In contrast, some prior art prostaglandin formulations have used salts such as sodium chloride to adjust tonicity to ophthalmically acceptable levels (e.g., 0.8 to 1.0 mg/ml NaCl equivalents). However, ionic tonicity adjusting agents reduce the solubility of the prostaglandin-related active. Thus, another advantage of the use of non-ionic tonicity adjusting agents (e.g., mannitol) in the present invention is the increased solubility of salts of the active agent.

Still another embodiment of the invention is a buffered 13,14-dihydro-15-keto-20-ethyl-PGF₂α isopropyl ester composition which offers improvements in manufacturing efficiency, improvements in shelf life and improvements in patient comfort. It is known that, for drops which are intended for direct instillation into the eye, a near neutral pH is preferred for patient comfort. In addition, adjustment of pH during manufacturing is difficult because of the small volume of solution in a consumer dispensing container. Further, the decomposition of 13,14-dihydro-15-keto-20-ethyl-PGF₂α isopropyl ester over time increases formulation acidity, and increased acidity causes an increase in the rate of 13,14-dihydro-15-keto-20-ethyl-PGF₂α isopropyl ester decomposition. Thus, the present embodiment is a 13,14-dihydro-15-keto-20-ethyl-PGF₂α isopropyl ester composition which is buffered sufficiently to maintain a pH of 4.5 to 8.0 (preferably 5 to 7.5, more preferably 6 to 7.5) over a period extending from manufacturing to about a year of shelf life, preferably 2 years of shelf life. Preferred ophthalmically acceptable buffers include EDTA, borates, citrates, lactates and phosphates.

Preferred compositions according to the invention include a composition comprising
(a) 0.06 to 0.24 weight percent isopropyl unoprostone;
(b) 0.3 to 2 weight percent of two non-ionic surfactant selected from the group consisting of CREMOPHOR RH, BRIJ 97, BRIJ 98, CREMOPHOR EL, Polysorbate 80 and mixtures thereof;
(c) 0.01 to 0.20 weight percent benzalkonium chloride;
(d) 0.01 to 0.1 weight percent EDTA;
(e) 0.10 to 10.0 weight percent mannitol;
(f) 0.01 to 0.05 molar of an ophthalmically acceptable buffer;
(g) an ophthalmically acceptable carrier;
in which the pH is adjusted to 4.5 to 8.0;
as well as a composition according to the invention, wherein the prostaglandin active agent (a) is 13,14-dihydro-15-keto-20-ethylPGF₂α isopropyl ester and present at a concentration of 0.001 to 0.30 weight percent; and the ophthalmic preservative (b) is present at a concentration of 0.005 to 0.2 weight percent.

The active agents useful in accordance with the invention may be selected from the group consisting of 13,14-dihydro-15-keto-20-ethyl-PGF₂α isopropyl ester, salts thereof, and mixture thereof, referred to herein as "prostaglandin active agents" or merely "active agent". Thus, the active agent is not limited by the specific form of the active, i.e., salt form. Rather, the prostaglandin active agent is active in that the agent causes a reduction of intraocular pressure (IOP) when applied to the ocular environment of a patient in need of reduction of intraocular pressure.

A group of prostaglandins which have been found to be useful in decreasing intraocular pressure are disclosed in U.S. Patent Nos. 4,599,353; 5,296,504; 5,422,368; and 5,578,618.

Prostaglandins of the present invention may be 13,14-dihydro-15-keto-20-ethyl-PGF₂α isopropyl ester salts. Suitable 13,14-dihydro-15-keto-20-ethyl-PGF₂α isopropyl ester salts are ophthalmically acceptable salts, including without limitation thereto, salts of alkali metals such as sodium or potassium; salts of an alkaline earth metal such as calcium or magnesium; salts of ammonia, methylamine, dimethylamine, cyclopentylamine, benzylamine, piperidine, monoethanolamine, diethanolamine, monomethylmonoethanolamine, tromethamine, lysine and tetralkylammonia; and the like and mixtures thereof.

A particularly preferred prostaglandin is isopropyl unoprostone or 13,14-dihydro-15-keto-20-ethyl PGF₂α isopropyl ester. The structure of isopropyl unoprostone is given below and a method of preparation is outlined in U.S. Patent 5,212,200,

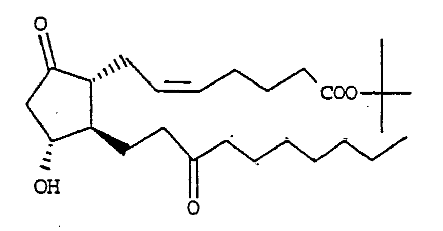

The preferred 13,14-dihydro-15-keto-20-ethyl-PGF₂α isopropyl ester concentration is an amount which will substantially reduce intraocular pressure (IOP) of an eye which has elevated IOP, especially in a patient suffering from glaucoma. Clearly the required concentration depends on a number of factors, including the efficacy of 13,14-dihydro-15-keto-20-ethyl-PGF₂α isopropyl ester in the presence of the other components, It has been determined that concentrations of active agents within the range of 0.001 to 0.30 weight percent are more efficacious for reducing intraocular pressure than concentrations above or below this range. In particular, a concentration of 0.06 to 0.24 weight percent active agent is preferred, while a concentration of 0.10 to 0.20 is more preferred. However, the preferred concentration in any specific application depends on a number of factors, such as the concentrations and chemical nature of other ingredients as well as the delivery method and conditions. Moreover, quite unexpectedly, further increases in active agent concentrations outside these preferred ranges may actually cause less of the desired decrease in intraocular pressure than the concentrations in the preferred ranges.

A surfactant, as used herein, refers to a surface active agent which improves the solubility of a substance, e.g. an active or drug, in a solvent. A non-ionic surfactant, as used herein, refers to a surfactant which possesses no easily ionizable groups.

U.S. Patent No. 5,208,256 discloses the use of Polysorbate 80 as a surfactant for prostaglandin-containing ophthalmic compositions. Polysorbate 80 improves the solubility of isopropyl unoprostone, so that a higher concentration of isopropyl unoprostone can be used in a solution form.

However, it has been discovered that while increasing the Polysorbate 80 concentration allows for increases in the prostaglandin concentration in solution, the preservative effectiveness decreases with increasing Polysorbate 80 concentrations. Moreover, it is desired to increase both the efficacy (e.g., by increasing the prostaglandin concentration) and preservative effectiveness of the known prostaglandin-containing ophthalmic formulations. Thus, it has been determined that use of more Polysorbate 80 has the disadvantage of decreasing preservative effectiveness, while less Polysorbate 80 has the disadvantage of reducing prostaglandin in solution and thereby reducing efficacy.

One embodiment of the present invention offers a solution to these problems by using a combination of two or more non-ionic surfactants. Certain combinations of non-ionic surfactants have been found to increase 13,14-dihydro-15-keto-20-ethyl-PGF₂α isopropyl ester active agent solubility without reducing preservative effectiveness as much as Polysorbate 80 alone in the same concentration.

A preferred group of non-ionic surfactants are those which exhibit better ophthalmic tolerance than Polysorbate 80 alone and/or which do not reduce preservative effectiveness or reduce preservative effectiveness less than Polysorbate 80 alone in the same concentration.

The first and second non-ionic surfactants may be selected from a group of non-ionic surfactants including, without limitation thereto, polyoxyethylene sorbitan fatty acid esters such as Polysorbates 20, 60 and 80; polyoxyethylene alkyl ethers such as Brij's (e.g., BRIJ 97 or BRIJ 98 from ICI Surfactants, Wilmington, Delaware), Cremophors (such as Cremophor RH or Cremophor EL), Volpo (e.g., VOLPO 10 and VOLPO 20 from Croda, Inc., Parsippany, New Jersey) and equivalents thereof. A preferred group includes polyoxyethylene 20 oleate (e.g., Polysorbate 80), Polyoxyl 10 oleyl ethers (e.g., Brij 97) and Polyoxyl 20 oleyl ethers (e.g., Brij 98).

A particularly preferred combination of surfactants is the combinations of a polyoxyethylene sorbitan fatty acid ester (especially Polysorbate 80) with a polyoxyethylene alkyl ethers (especially BRIJ 97 or BRIJ 98).

Thus, use of at least two surfactants together provides an unexpected synergistic result in that the total concentration of surfactant required to achieve a desired 13,14-dihydro-15-keto-20-ethyl-PGF₂α isopropyl ester active agent solubility is less that the concentration required for an individual surfactant. In addition, certain combinations of surfactants actually improve the preservative effectiveness. Specifically, the combination of Polysorbate 80 with a BRIJ surfactant improves BAK preservative effectiveness relative to the same concentration of Polysorbate 80 alone. Furthermore, this combination of surfactants improves the emulsion stability of the formulation.

The total concentration of surfactant used depends, in large part, on the solubilizing character of the particular surfactant or surfactants and the concentration and chemical nature of the particular active agent which the surfactant is intended to solubilize. In general, the total surfactant concentration may range from 0.1 to 5 weight percent. A preferred surfactant concentration is 0.3 to 2.0 weight percent. More preferably, the surfactant concentration is 0.5 to 1.5 weight percent.

A "preservative", as used herein, refers to an additive which inhibits both microbial growth and kills microorganisms which inadvertently contaminate the ophthalmic solution upon exposure to the surroundings. The preservative may be selected from a variety of well known preservatives, including hydrophobic or non-charged preservatives, anionic preservatives, and cationic preservatives. A "preservative enhancing agent", as used herein, refers to an additive which increases the preservative effectiveness of a preservative, or the preservative effectiveness of a preserved formulation, but which would not typically be used solely to preserve an ophthalmic formulation.

Strong preservatives include cationic preservatives which include, without limitation thereto, polymyxin B sulfate, quaternary ammonium compounds, poly(quatemary ammonium) compounds, p-hydroxybenzoic acid esters, certain phenols and substituted alcohols, benzalkonium chloride, benzoxonium chloride, cetylpridinium chloride, benzethonium chloride, cetyltrimethyl ammonium bromide, chlorhexidine, poly(hexamethylene biguanide), and mixtures thereof. Poly(quaternary ammonium) compounds include BUSAN 77, ONAMER M, MIRAPOL A15, IONENES A, POLYQUATERNIUM 11, POLYQUATERNIUM 7, BRADOSOL, AND POLYQUAT D-17-1742. A preferred preservative for the ophthalmic field is benzalkonium chloride.

Anionic preservatives include, without limitation thereto, 1-octane sulfonic acid (monosodium salt); 9-octadecenoic acid (sulfonated); ciprofloxacin; dodecyl diphenyloxide-disulfonic acid; ammonium, potassium, or sodium salts of dodecyl benzene sulfonic acid; sodium salts of fatty acids or tall oil; naphthalene sulfonic acid; sodium salts of sulfonated oleic acid; organic mercurials such as thimerosal (sodium ethylmercurithiosalicylate); thimerfonate sodium (sodium p-ethylmercurithiophenylsulfonate).

Hydrophobic or non-ionic preservatives include, without limitation thereto, 2,3-dichloro-1,4-naphthoquinone; 3-methyl-4-chlorophenol (PREVENTOL CMK); 8-hydroxyquinoline and derivatives thereof; benzyl alcohol; bis(hydroxyphenyl) alkanes; bisphenols; chlorobutanol; chloroxylenol; dichlorophen [2,2'-methylene-bis(4-chlorophenol)] (PANACIDE); ortho-alkyl derivatives of para-bromophenol and para-chlorophenol; oxyquinoline; para-alkyl derivatives of ortho-chlorophenol and ortho-bromophenol; pentachlorophenyl laurate (MYSTOX LPL); phenolic derivatives such as 2-phenylphenol, 2-benzyl-4-chlorophenol, 2-cyclopentyl-4-chlorophenol, 4-t-amylphenol, 4-t-butylphenol, and 4- and 6-chloro-2-pentylphenol; phenoxy fatty acid polyester (PREVENTOL B2); phenoxyethanol; and phenylethyl alcohol.

In one embodiment, the preservative is present in the solution in an amount sufficient to kill microbes which may inadvertently enter the dispensing container over the period of use. The desirable concentration will depend on a number of factors, including the strength of the preservative, the conditions of dispenser use, and the length of time the dispenser and solution will be in service. Generally, the strong preservative may be present in a concentration from 0.00005 to 0.2 weight percent, more preferably the concentration is 0.005 to 0.2 weight percent, and even more preferably, the strong preservative concentration is 0.01 to 0.015 weight percent.

Preservative enhancers are understood as an ophthalmically acceptable agent which enhances the effectiveness of the preservative may be advantageously added to the formulation. Examples of preservative enhancing agents useful in accordance with the present invention include, without limitation thereto, chelating agents such as ethylene diamine tetraacetate (EDTA), derivatives thereof, salts thereof and mixtures thereof.

The preservative enhancing agent is intended to overcome any remaining microbial burden which the strong preservative did not. For example, while BAK kills nearly all *Pseudomonas,* there may remain some resistant strain or strains, which may propagate over time. Thus, it is desirable to add a preservative enhancing agent, such as EDTA, to kill the remaining BAK-resistant *Pseudomonas.* It is believed that EDTA destroys the *Pseudomonas* by chelation with Ca⁺ ions. Accordingly, a preferred class of weak preservatives are chelating agents, especially calcium chelating agents.

The use of EDTA is particularly preferred in part because EDTA prevents the growth of BAK-resistant *Pseudomonas.* However, EDTA has also been found to have advantages in addition to its preservative enhancing function. EDTA can be used to buffer the formulation to achieve the desired pH. Further, EDTA may provide a stabilization function for the active agent, thereby inhibiting degradation and increasing shelf life.

The concentration of preservative enhancing agent which is preferred will depend on a number of factors, such as the efficacy of the strong preservative at the chosen concentration and the preservative enhancing effectiveness of the preservative enhancing agent. The concentration of preservative enhancer should be high enough to deactivate amounts of *Pseudomonas* which are dangerous to the patient, but the concentration should be low enough to avoid any substantial ocular discomfort.

If a chelating agent such as EDTA is used, a concentration of 0.01 to 0.1 weight percent is preferred. More preferably, the concentration is 0.03% to 0.07%.

Another additive which was determined, quite unexpectedly, to enhance the preservative effectiveness of formulations containing active agents is mannitol. It is known to use mannitol to adjust tonicity of an solution to improve ophthalmic compatibility, e.g., by adjusting to nearly an isotonic state. However, the preservative enhancing effect was unexpectedly found in formulations containing active agents. In general, it is believed that other non-ionic tonicity adjusting agents, especially other simple sugars, may perform the same function.

Thus, use of one or more preservative enhancers can provide at least two advantages. First, the amount of strong preservative, which may cause irritation to some patients, required for a given level of preservation is reduced. Second, the preservative enhancers may be chosen so that they serve functions in addition to improving preservation of the formulation.

An additional weaker preservative may be added to the container. The weaker preservative, at the concentrations of use, should not be sufficiently potent to cause irritation of the target tissue which the solution will contact. Examples of weaker preservatives useful in accordance with the present invention include, without limitation thereto, peroxides, such as hydrogen peroxide; peroxide-generating species, such as an alkali perborate or a combination of sodium perborate, boric acid, and sodium borate; urea peroxide; sodium peroxide carbonate; sodium persulfate; sodium perphosphate; and poly(vinyl pyrrolidone) hydrogen peroxide. A preferred weak preservative is a perborate such as sodium perborate.

If a peroxide or peroxide-generating species is used, the peroxide concentration should be less than 0.1 weight percent, preferably 0.004 to 0.05 weight percent, more preferably 0.001 to 0.02 weight percent.

The addition of a buffer offers at least two advantages. First, the buffer helps maintain the pH of the formulation at an ophthalmically acceptable level for instillation directly into the eye. Second, incorporating a buffer early in the manufacturing process reduces the complexity of controlling the pH during manufacturing.

A variety of ophthalmically acceptable buffers may be used. For example, borate buffers such as a combination of boric acid and sodium borate, phosphate buffers, citrates, lactates, equivalents thereof and mixtures thereof.

Also, as mentioned earlier EDTA, which is a preferred weak preservative, may serve a buffering function. Thus, EDTA may advantageously be used to serve at least two functions, i.e., to adjust and maintain the pH and to act as a preservative enhancer. It should be noted that EDTA may further serve as a stabilizer for the active agent, i.e., inhibiting degradation of the active agent (e.g., by chelating metal ions which may catalyze degradation or acting as an antioxidant).

The compositions according to the invention comprise non-ionic tonicity adjusting agents in order to maximize the solubility of 13,14-dihydro-15-keto-20-ethyl-PGF₂α isopropyl ester. Examples of useful non-ionic tonicity adjusting agents include mannitol, sorbitol, glycerol, polyethylene glycols (PEG), polypropylene glycols (PPG), sorbitol and mixtures thereof. A preferred non-ionic tonicity adjusting agent is mannitol.

In addition to the unexpected enhancement of preservative effectiveness, certain non-ionic tonicity adjusting agents may serves additional functions in ophthalmic formulations containing active agents. For example, it has been unexpectedly discovered that mannitol increases the solubility of isopropyl unoprostone, a preferred active agent. Thus, use of appropriate non-ionic tonicity adjusting agents can (1) result in lower requirements for strong preservatives, which may cause ocular irritation, (2) reduce the concentration of solubility enhancers and/or reduce the amount of active agent required to achieve a chosen active concentration in solution, and (3) adjust the tonicity to ophthalmically acceptable levels.

The tonicity adjusting agent concentration is typically determined by adding sufficient tonicity adjusting agent to produce a formulation with is substantially isotonic, in order to maximize patient comfort. An isotonic solution is one which may be expressed as having a concentration equivalent to 0.9 mg/ml sodium chloride in deionized water. Substantially isotonic, as used herein, refers to a formulation having 0.8 to 1.0 mg/ml NaCl equivalents.

In order to achieve a substantially isotonic solution, 0.1 to 10 weight percent of non-ionic tonicity adjusting agent should be added to the formulation. More preferably, the formulation will include 1 to 7 weight percent of non-ionic tonicity adjusting agent. Even more preferably, the formulation will include 3 to 5 weight percent of non-ionic tonicity adjusting agent.

A preferred carrier or solvent for the present invention is water, for example, in the form of distilled water or physiological saline. However, the invention is not limited to a particular solvent or diluent, except that the solvent must be ophthalmically compatible under the conditions of intended use. Other examples of diluents for producing a non-aqueous suspension include, without limitation thereto, edible oils, liquid paraffins, mineral oil, propylene glycol, p-octyldodecanol, mixtures thereof and the like.

While the 13,14-dihydro-15-keto-20-ethyl-PGF₂α isopropyl ester formulations described herein are useful in treating ocular hypertension without additional actives, additional actives may be desirable and are within the scope of the invention. For example, the present formulations may include conventional cholinergic ocular hypertensive agents such as pilocarpin or carbachol; anticholinesterases such as demecarium, D.F.P. or echothiophate; miotics such as physostigmine salicylate or pilocarpine hydrochloride; and antiinflammatories such as diclofenac, penicillin, sulfonamide, chloramphenicol, cortisone or chlorpheniramine.

The aforementioned actives are listed to further the reader's understanding of the various embodiments of the invention. Thus, the list of actives, provided above for addition to the present formulations, is not exhaustive and the invention is not so limited.

The present ophthalmic compositions may be applied to the ocular tissue or ocular fluids via a number of techniques. For example, a solution or slurry of the ophthalmic composition may be directly instilled into the eye in a droplet, spray or mist form. Alternatively, a drug delivery device with a reservoir (e.g., a polymeric network), which holds the ophthalmic composition, may be inserted into the ocular cavity (e.g., under the eyelid) and left for an extended period of time. The compositions may also be applied transdermally, including by electrotransport, preferably to skin areas near the eye. Injection, either subcutaneous or intraocular, and oral administration may also be useful delivery routes.

However, application of the ophthalmic compositions to the ocular fluids by dropwise addition is currently a preferred method. The number of drops and number of applications per day may vary, depending, *inter alia,* on the composition efficacy, patient tolerance and relative state of the disease.

Thus, one embodiment of using the compositions of the invention is a method of reducing ocular hypertension, which involves administering to the ocular fluids or ocular tissue an ophthalmic composition including a active agent which is selected from the group consisting of 13,14-dihydro-15-keto-20-ethyl-PGF₂α isopropyl ester salts thereof, and mixtures thereof; an ophthalmic preservative; a non-ionic tonicity adjusting agent; and an ophthalmically acceptable carrier. The non-ionic tonicity adjusting agent is preferably present in a concentration sufficient both to adjust the tonicity of the composition and to increase the preservative effectiveness. The composition is effective in lowering intraocular pressure when administered to a patient in need of a reduction in intraocular pressure.

Another embodiment is a method of reducing ocular hypertension, which includes administering to the ocular fluids or ocular tissue an ophthalmic composition including a 13,14-dihydro-15-keto-20-ethyl-PGF₂α isopropyl ester; a first non-ionic surfactant; a second non-ionic surfactant; and an ophthalmically acceptable carrier. The total surfactant concentration is lower than the surfactant concentration which would be required to solubilize the active agent for either individual non-ionic surfactant.

Yet another embodiment is a method of reducing ocular hypertension, which includes administering to the ocular fluids or ocular tissue an ophthalmic composition including a 13,14-dihydro-15-keto-20-ethyl-PGF₂α isopropyl ester; a strong preservative; a non-ionic surfactant which increases solubility of the active agent but decreases the preservative effectiveness of the strong preservative; a preservative enhancer which increases the effectiveness of the strong preservative; and an ophthalmically acceptable carrier.

The previous disclosure will enable one having ordinary skill in the art to practice the invention. In order to better enable the reader to understand specific embodiments and the advantages thereof, reference to the following examples is suggested.

### EXAMPLE 1

A 0.12% isopropyl unoprostone ophthalmic formulation was prepared in accordance with the following procedure. A surfactant solution was prepared by dissolving 0.517 grams of Polysorbate-80 and 0.221 grams of Brij-97 were dissolved in 70 grams of distilled water. The surfactant solution was added to 0.132 grams of isopropyl unoprostone (Ueno Fine Chemicals, Osaka, Japan) and stirred overnight. 1.034 grams of 1.06 weight percent benzalkonium chloride (BAK) solution, 11.0 grams of 0.01 molar phosphate buffer, and 0.011 grams of ethylenediamine tetraacetate (EDTA) were added to the isopropyl unoprostone solution and mixed until dissolved. Distilled water was added to the resultant solution to bring the weight up to 90% of the final desired weight (110 grams). 5.153 grams of mannitol was added to the solution, with stirring until dissolved. Finally, distilled water was added to bring the solution to a final weight of 110 grams.

The resultant solution had a composition, based on weight, of:
0.12% isopropyl unoprostone,
0.47% Polysorbate 80,
0.20% Brij 97,
0.011 % BAK,
0.01 % EDTA, and
4.7% mannitol.

A clear solution was observed. Accordingly, the surfactants, which had a total weight percentage of 0.67%, completely solubilized the isopropyl unoprostone.

30 microliters of the formulation were instilled into the eye of a rabbit at time designated as t=0. The intraocular pressure (IOP) was measured at t=0, 30, 60, 120, 180, 240, 300, and 360 minutes after instillation. IOP was measured by pneumatonometry. IOP, expressed as an average of the samples studied and as a percentage of the t=0 pressure, is shown in TABLE I.

### EXAMPLE 2

An isopropyl unoprostone ophthalmic formulation was prepared substantially in accordance with the procedure of Example 1, with the exceptions being that formulation included, in weight percentages: 0.18% isopropyl unoprostone, 0.70% Polysorbate 80 and 0.30% Brij 97. Furthermore, no phosphate buffer was necessary, but pH was adjusted with NaOH. The isopropyl unoprostone was completely solubilized as in Example 1.

The IOP lowering effect of this formulation was tested substantially in accordance with the procedure described in Example 1. Intraocular pressure, expressed as a percentage of the t=0 pressure, is shown in TABLE I.

### EXAMPLE 3

An isopropyl unoprostone ophthalmic formulation was prepared substantially in accordance with the procedure of Example 1, with the exceptions being that formulation included, in weight percentages: 0.24% isopropyl unoprostone, 0.95% Polysorbate 80 and 0.42% Brij 97. The isopropyl unoprostone was completely solubilized as in Example 1.

The IOP lowering effect of this formulation was tested substantially in accordance with the procedure described in Example 1. Intraocular pressure, expressed as a percentage of the t=0 pressure, is shown in TABLE I.

Examination of the data generated from the Examples 1-3 shows that a 0.18% isopropyl unoprostone is more effective than 0.12% or a 0.24% isopropyl unoprostone formulations. Accordingly, a preferred range of isopropyl unoprostone concentrations is 0.12% to 0.24%. An even more preferred concentration range of isopropyl unoprostone is 0.18%.

**TABLE I**

| IOP as a percentage of the initial IOP | | | |
|---|---|---|---|
| Time after instillation | **Example 1** 0.12% isopropyl unoprostone | **Example 2** 0.18% isopropyl unoprostone | **Example 3** 0.24% isopropyl unoprostone |
| 0 | 100 | 100 | 100 |
| 30 | 105 | 110 | 111 |
| 60 | 99 | 99 | 110 |
| 120 | 88 | 67 | 82 |
| 180 | 93 | 56 | 80 |
| 240 | 108 | 67 | 84 |
| 300 | 109 | 79 | 83 |
| 360 | 110 | 86 | 92 |

### EXAMPLE 4

An isopropyl unoprostone ophthalmic formulation was prepared substantially in accordance with the procedure of Example 1, with modifications of the relative concentration of the components. The resultant formulation in weight percentages was:
0.12% isopropyl unoprostone,
0.47% Polysorbate 80,
0.20% Brij 97,
0.010% BAK,
0.01% EDTA, and
4.4% mannitol.

Thus, the total surfactant concentration was 0.67%. The isopropyl unoprostone was completely solubilized as in Example 1. Comparative results are presented in Table II.

### EXAMPLE 5

An isopropyl unoprostone ophthalmic formulation was prepared substantially in accordance with the procedure of Example 2, with modifications of the relative concentration of the components, including a substitution of Volpo 10 for Brij 97. The resultant formulation in weight percentages was:
0.12% isopropyl unoprostone,
0.47% Polysorbate 80,
0.20% Volpo 10,
0.013% BAK,
0.05% EDTA, and
4.3% mannitol.

Thus, the total surfactant concentration was 0.67%. The isopropyl unoprostone was completely solubilized as in Example 1. Comparative results are presented in Table II.

### EXAMPLE 6

A 0.12% isopropyl unoprostone ophthalmic formulation was prepared in accordance with the following procedure. 6 grams of sodium chloride and 0.2 grams of benzalkonium chloride were dissolved in about a liter of distilled water. 0.12 grams of isopropyl unoprostone and about one (1) gram of Polysorbate 80 were mixed into the BAK solution. The resultant formulation in weight percentages included:
0.12% isopropyl unoprostone,
1.0% Polysorbate 80,
0.020% BAK,
0.6% sodium chloride

Thus, the total surfactant concentration was 1.0%. The isopropyl unoprostone was solubilized, i.e., the solution appeared clear. Comparative results are presented in Table II.

### EXAMPLE 7

A 0.12% isopropyl unoprostone ophthalmic formulation was prepared substantially in accordance with Example 6, with the exception being that a reduced amount of Polysorbate 80 was used. The resultant formulation included:
0.12% isopropyl unoprostone,
0.85% Polysorbate 80,
0.020% BAK,
0.6% sodium chloride

Thus, the total surfactant concentration was 0.85%. The isopropyl unoprostone was solubilized, i.e., the solution appeared clear. Comparative results are presented in Table II.

### EXAMPLE 8

A 0.12% isopropyl unoprostone ophthalmic formulation was prepared substantially in accordance with Example 7, with the exception being that a reduced amount of Polysorbate 80 was used. The resultant formulation included:
0.12% isopropyl unoprostone,
0.80% Polysorbate 80,
0.020% BAK,
0.6% sodium chloride

Thus, the total surfactant concentration was 0.80%. The isopropyl unoprostone was not completely solubilized, i.e., the solution appeared cloudy. Comparative results are presented in Table II.

The isopropyl unoprostone was not completely solubilized, i.e., phase separation was observed. Comparative results are presented in Table II.

**TABLE II**

| | Example 1 | Example 4 | Example 5 | Example 6 | Example 7 | Example 8 |
|---|---|---|---|---|---|---|
| % isopropyl unoprostone | 0.12 | 0.12 | 0.12 | 0.12 | 0.12 | 0.12 |
| % Polysorbate 80 | 0.47 | 0.47 | 0.47 | 1.0 | 0.85 | 0.80 |
| % Brij 97 | 0.20 | 0.20 | -- | -- | -- | -- |
| % Volpo 10 | -- | -- | 0.20 | -- | -- | -- |
| Total % surfactant | 0.67 | 0.67 | 0.67 | 1.0 | 0.85 | 0.80 |
| Solubility | complete | complete | complete | complete | complete | incomplete cloudy formulation |

Examples 1 and 4-8, along with Table II, show that the combination of Polysorbate 80 with Brij 97 or Volpo 10 solubilizes isopropyl unoprostone better than Polysorbate 80 alone. In Example 8, a 0.80% total surfactant formulation with Polysorbate 80 alone did not adequately solubilize the active, while a 0.67% total surfactant formulation with the combination of surfactants provided complete solubility. Thus, a lower total surfactant concentration may be achieved by using two or more surfactants rather than one surfactant in a formuation containing prostaglandin active agents.

### EXAMPLE 9

a 100 gram ophthlamic formulation including 0.12% isopropyl unoprostone was prepared in accordance with the following procedure. 0.12 grams isopropyl unoprostone and 1.0 grams Polysorbate 80 were added to a beaker, followed by about 90 grams distilled water. The mixture was stirred until dissolved. 1.2 grams of an 1% BAK solution and 0.05 grams EDTA were added to the resultant solution. 3.3 grams of mannitol was added with mixing until dissolution was achieved.

The resultant formulation contained:
0.12% isopropyl unoprostone
1.0% Polysorbate 80
0.012% BAK
0.05% EDTA
3.3% mannitol

The formulation was subject to standard U.S. Pharmacopia and European Pharmacopia Criteria "A" and "B" preservative effectiveness testing. The formulation passed all three tests. Results are summarized in Table III.

### EXAMPLE 10

A formulation was prepared substantially in accordance with the procedure described in Example 9, except that sodium chloride was used as the tonicity adjusting agent instead of mannitol. The formulation had the following composition:
0.12% isopropyl unoprostone
1.0% Polysorbate 80
0.012% BAK
0.05% EDTA
0.6% sodium chloride

The formulation failed the European Pharmacopia Criteria "A" and "B" preservative effectiveness tests, while passing the USP test. Results are summarized in Table III.

### EXAMPLE 11

A formulation was prepared substantially in accordance with the procedure described in Example 9, except that sodium chloride was used as the tonicity adjusting agent instead of mannitol and additional BAK and EDTA were used. The formulation had the following composition:
0.12% isopropyl unoprostone
1.0% Polysorbate 80
0.013% BAK
0.10% EDTA
0.6% sodium chloride

The formulation failed the European Pharmacopia Criteria "A" and "B" preservative effectiveness tests, while passing the USP test. Results are summarized in Table III.

### EXAMPLE 12

A formulation was prepared substantially in accordance with the procedure described in Example 9, except that additional BAK and EDTA were used as compared with Example 9. The formulation had the following composition:
0.12% isopropyl unoprostone
1.0% Polysorbate 80
0.013% BAK
0.10% EDTA
3.3% mannitol

The formulation passed the European Pharmacopia Criteria "A" and "B" tests as well as the USP test. Results are summarized in Table III.

### EXAMPLE 13

A formulation was prepared substantially in accordance with the procedure described in Example 12, except that sodium chloride was substituted for mannitol and additional BAK was used as compared with Example 12. The formulation had the following composition:
0.12% isopropyl unoprostone
1.0% Polysorbate 80
0.014% BAK
0.10% EDTA
0.6% sodium chloride

The formulation failed the European Pharmacopia Criteria "A" and "B" preservative effectiveness tests, while passing the USP test. Results are summarized in Table III.

### EXAMPLE 14

A formulation was prepared substantially in accordance with the procedure described in Example 13, except that additional BAK was used as compared with Example 13. The formulation had the following composition:
0.12% isopropyl unoprostone
1.0% Polysorbate 80
0.015% BAK
0.10% EDTA
0.6% sodium chloride

The formulation failed the European Pharmacopia Criteria "A" (EPA) and "B" (EPB) preservative effectiveness tests, while passing the US-Pharmacopia (USP) test. Results are summarized in Table III.

**TABLE III**

| | Example 9 | Example 10 | Example 11 | Example 12 | Example 13 | Example 14 |
|---|---|---|---|---|---|---|
| % isopropyl unoprostone | 0.12 | 0.12 | 0.12 | 0.12 | 0.12 | 0.12 |
| % Polysorbate 80 | 1.0 | 1.0 | 1.0 | 1.0 | 1.0 | 1.0 |
| % BAK | 0.012 | 0.012 | 0.013 | 0.013 | 0.014 | 0.015 |
| % EDTA | 0.05 | 0.05 | 0.10 | 0.10 | 0.10 | 0.10 |
| % mannitol | 3.3 | -- | -- | 3.3 | -- | -- |
| % sodium chloride | -- | 0.6 | 0.6 | -- | 0.6 | 0.6 |
| EPA | PASS | fail | fail | PASS | fail | fail |
| EPB | PASS | fail | fail | PASS | fail | fail |
| USP | PASS | PASS | PASS | PASS | PASS | PASS |

Examples 9-14 and Table III illustrate that the non-ionic tonicity adjusting agent mannitol enhances preservative effectiveness as compared to the ionic tonicity adjusting agent sodium chloride.

## Claims

1. A composition, comprising:
(a) a prostaglandin active agent which is a 13,14-dihydro-15-keto-20-ethyl-PGF₂α isopropyl ester
salts thereof, and mixtures thereof;
(b) an ophthalmic preservative;
(c) a non-ionic tonicity adjusting agent, wherein said non-ionic tonicity adjusting agent is present in a concentration sufficient to:
(i) adjust the tonicity of the composition and
(ii) increase the preservative effectiveness;
(d) an ophthalmically acceptable carrier, and
(e) further comprising 0.1 to 5.0 weight percent of a non-ionic surfactant selected from the group consisting of polyoxyethylene sorbitan fatty acid esters, polyoxyethylene alkyl ethers, and mixtures thereof.

2. A composition of claim 1, further comprising a chelating agent.

3. A composition of claim 2, wherein said chelating agent is ethylenediamine tetraacetic acid or a salt thereof.

4. A composition of claim 2, comprising 0.1 to 10 weight percent non-ionic tonicity adjusting agent and 0.01 to 0.10 weight percent chelating agent.

5. A composition of claim 1, comprising 0.001 to 0.30 weight percent of the active agent.

6. A composition of claim 5, comprising 0.06 to 0.24 weight percent of the active agent.

7. A composition of claim 6, comprising 0.10 to 0.20 weight percent of the active agent.

8. A composition of claim 1, wherein the non-ionic surfactant is Polysorbate 80 and is present at a concentration of 0.3 to 2.0 weight percent.

9. A composition of claim 1, comprising 0.001 to 0.05 weight percent of an ophthalmic preservative selected from the group consisting of polymyxin B sulfate; quaternary ammonium compounds; poly(quaternary ammonium) compounds; p-hydroxybenzoic acid esters; certain phenols and substituted alcohols; benzalkonium chloride; benzoxonium chloride; cetylpridinium chloride; benzethonium chloride; cetyltrimethyl ammonium bromide; chlorhexidine, poly(hexamethylene biguanide); 1-octane sulfonic acid (monosodium salt); 9-octadecenoic acid (sulfonated); ciprofloxacin; dodecyl diphenyloxide-disulfonic acid; ammonium, potassium, or sodium salts of dodecyl benzene sulfonic acid; sodium salts of fatty acids or tall oil; naphthalene sulfonic acid; sodium salts of sulfonated oleic acid; organic mercurials such as thimerosal; thimerfonate sodium; 2,3-dichloro-1,4-naphthoquinone; 3-methyl-4-chlorophenol; 8-hydroxyquinoline; benzyl alcohol; bis(hydroxyphenyl) alkanes; bisphenols; chlorobutanol; chloroxylenol; dichlorophen [2,2'-methylene-bis(4-chlorophenol)]; ortho-alkyl derivatives of para-bromophenol and para-chlorophenol; oxyquinoline; para-alkyl derivatives of ortho-chlorophenol and ortho-bromophenol; pentachlorophenyl laurate; phenolic derivatives such as 2-phenylphenol, 2-benzyl-4-chlorophenol, 2-cyclopentyl-4-chlorophenol, 4-t-amylphenol, 4-t-butylphenol, and 4- and 6-chloro-2-pentylphenol; phenoxy fatty acid polyester; phenoxyethanol; phenylethyl alcohol; derivatives and mixtures thereof.

10. A composition of claim 1, further comprising a weak preservative selected from the group consisting of peroxides, peroxide-generating species and mixtures thereof.

11. A composition of claim 1, further comprising an ophthalmically acceptable buffer selected from the group consisting of EDTA, borates and phosphates, wherein said buffer is present in an amount sufficient to maintain the composition at a pH of 4.5 to 8.0 over a period of up to one year in storage.

12. A composition of claim 1, comprising 0.1 to 10 weight percent of an ophthalmically acceptable non-ionic tonicity adjusting agent selected from the group consisting of mannitol, sorbitol, glycerol, polyethylene glycols (PEG), polypropylene glycols (PPG), and mixtures thereof.

13. A composition of claim 5, wherein the ophthalmic preservative (b) is present at a concentration of 0.005 to 0.2 weight percent.

14. A composition of claim 1 comprising:
(1) a prostaglandin active agent;
(2) a first non-ionic surfactant; and
(3) a second non-ionic surfactant; and
(4) an ophthalmically acceptable carrier,
wherein the total surfactant concentration is lower than the surfactant concentration which would be required to solubilize the prostaglandin active agent for either individual non-ionic surfactant.

15. A composition of claim 14, wherein said first non-ionic surfactant is a polyoxyethylene sorbitan fatty acid ester and said second non-ionic surfactant is a polyoxyethylene alkyl ether.

16. A composition of claim 15, wherein said first non-ionic surfactant is a Polysorbate compound and said second non-ionic surfactant is Brij compound.

17. A composition of claim 16, wherein the total non-ionic surfactant concentration in the composition is 0.3 to 2.0 weight percent.

18. A composition of claim 1, wherein said preservative (b) is a strong preservative; and said non-ionic surfactant (e) increases solubility of the prostaglandin active agent but decreases the preservative effectiveness of the strong preservative; which composition further comprises
(g) a preservative enhancer which increases the effectiveness of the strong preservative.

19. A composition of claim 18, wherein said preservative enhancer is selected from the group consisting of mannitol, ethylenediamine tetraacetic acid, salts thereof, and mixtures thereof.

20. A composition of claim 19, wherein said preservative enhancer is mannitol.

21. A composition of claim 19, wherein said preservative enhancer is ethylenediamine tetraacetic acid.

22. A composition of claim 21, wherein said preservative enhancer is a mixture of ethylenediamine tetraacetic acid and mannitol.

23. Use of a composition in accordance to any of the preceding claims in the preparation of an ophthalmic composition in the treatment of ocular hypertension.

## Patentansprüche

1. Zusammensetzung, umfassend
(a) einen Prostaglandinwirkstoff, bei dem es sich um einen 13,14-Dihydro-15-keto-20-ethyl-PGF₂α-isopropylester, Salze hiervon und Gemische hiervon handelt,
(b) ein ophthalmisches Konservierungsmittel,
(c) ein nichtionisches Tonizitätseinstellmittel, wobei dieses nichtionische Tonizitätseinstellmittel in einer Konzentration vorhanden ist, die ausreicht zur
(i) Einstellung der Tonizität der Zusammensetzung und
(ii) Erhöhung der Wirksamkeit des Konservierungsmittels,
(d) einen ophthalmisch annehmbaren Träger und
(e) ferner 0.1 bis 5,0 Gew.-% eines nichtionischen Tensids, das ausgewählt ist aus der Gruppe, die besteht aus Polyoxyethylensorbitanfettsäureestern, Polyoxyethylenalkylethern und Gemischen hiervon.

2. Zusammensetzung nach Anspruch 1, weiter umfassend einen Chelatbildner.

3. Zusammensetzung nach Anspruch 2, worin der Chelatbildner Ethylendiamintetraessigsaure oder ein Salz hiervon ist.

4. Zusammensetzung nach Anspruch 2, umfassend 0,1 bis 10 Gew.-% nichtionisches Tonizitätseinstellmittell und 0,01 bis 0,10 Gew.-% Chelatbildner.

5. Zusammensetzung nach Anspruch 1, umfassend 0,001 bis 0,30 Gew.-% Wirkstoff.

6. Zusammensetzung nach Anspruch 5, umfassend 0,06 bis 0,24 Gew-% Wirkstoff.

7. Zusammensetzung nach Anspruch 6, umfassend 0,10 bis 0,20 Gew-% Wirkstoff.

8. Zusammensetzung nach Anspruch 1, worin das nichtionische Tensid Polysorbat 80 ist und in einer Konzentration von 0,3 bis 2,0 Gew.-% vorhanden ist.

9. Zusammensetzung nach Anspruch 1, umfassend 0,001 bis 0,05 Gew.-% ophthalmisches Konservierungsmittel, das ausgewählt ist aus der Gruppe, die besteht aus Polymyxin-B-sulfat, quartären Ammoniumverbindungen. Poly(quartären-ammonium)verbindungen, p-Hydroxybenzoesäureestem, bestimmten Phenolen, und substituierten Alkoholen, Benzalkoniumchlorid, Benzoxoniumchlorid, Cetylpyridiniumchlorid, Benzethoniumchlorid, Cetyltrimethylammoniumbromid, Chlorhexidin, Poly(hexamethylenbiguanid), 1-Octansulfonsäure (Mononatriumsalz), 9-Octadecensäure (sulfoniert), Ciprofloxacin, Dodecyldiphenyloxiddisulfonsäure, Ammonium-, Kalium- oder Natriumsalzen von Dodecylbenzolsulfonsäure, Natriumsalzen von Fettsäuren oder Tallöl, Naphthalinsulfonsäure, Natriumsalzen von sulfonierter Ölsäure, organischen Quecksilberverbindungen, wie Thimerosal, Thimerfonatnatrium, 2,3-Dichlor-1,4-naphthochinon, 3-Methyl-4-chlorphenol, 8-Hydroxychinolin, Benzylalkohol, Bis(hydroxyphenyl)alkanen, Bisphenolen, Chlorbutanol, Chlorxylenol, Dichlorphen [2,2'-Methylen-bis(4-chlorpheno)], ortho-Alkylderivaten von para-Bromphenol und para-Chlorphenol, Oxychinolin, para-Alkylderivaten von ortho-Chlorphenol und ortho-Bromphenol, Pentachlorphenyllaurat, Phenolderivaten, wie 2-Phenylphenol, 2-Benzyl-4-chlorphenol, 2-Cyclopentyl-4-chlorphenol, 4-t-Amylphenol, 4-t-Butylphenol und 4- und 6-Chlor-2-pentylphenol, Phenoxylettsäurepolyestern, Phenoxyethanol, Phenylethylalkohol, Derivaten und Gemischen hiervon.

10. Zusammensetzung nach Anspruch 1, weiter umfassend ein schwaches Konservierungsmittel, das ausgewählt ist aus der Gruppe, die besteht aus Peroxiden, Peroxid-bildenden Spezies und Gemischen hiervon.

11. Zusammensetzung nach Anspruch 1, weiter umfassend einen ophthalmisch annehmbaren Puffer, der ausgewählt ist aus der Gruppe, die besteht aus EDTA, Boraten und Phosphaten, worin dieser Puffer in einer solchen Menge vorhanden ist, dass die Zusammensetzung während einer Aufbewahrungsdauer bis zu 1 Jahr auf einem pH Wert von 4,5 bis 8,0 gehalten wird.

12. Zusammensetzung nach Anspruch 1, umfassend 0,1 bis 10 Gew.-% eines ophthalmisch annehmbaren nichtionischen Tonizitätseinstellmittels, das ausgewählt ist aus der Gruppe, die besteht aus Mannit, Sorbit, Glycerin, Polyethylenglycolen (PEG), Polypropylenglycolen (PPG) und Gemischen hiervon.

13. Zusammensetzung nach Anspruch 5, worin das ophthalmische Konservierungsmittel (b) in einer Konzentration von 0,005 bis 0,2 Gew.-% anwesend ist.

14. Zusammensetzung nach Anspruch 1, umfassend
(1) einen Prostaglandinwirkstoff,
(2) ein erstes nichtionisches Tensid,
(3) ein zweites nichtionisches Tensid und
(4) einen ophthalmisch annehmbaren Träger,
worin die gesamte Tensidkonzentration niedriger ist als die Tensidkonzentration, die erforderlich wäre, um den Prostaglandinwirkstoff jeweils in jedem der beiden nichtionischen Tenside zu solubilisieren.

15. Zusammensetzung nach Anspruch 14, worin das erste nichtionische Tensid ein Polyoxyethylensorbitanfettsäureester ist und das zweite nichtionische Tensid ein Polyoxyethylenalkylether ist

16. Zusammensetzung nach Anspruch 15, worin das erste nichtionische Tensid eine Polysorbatverbindung ist und das zweite nichtionische Tensid eine Brijverbindung ist.

17. Zusammensetzung nach Anspruch 16, worin die Gesamtkonzentratton an nichtionischem Tensid in der Zusammensetzung 0,3 bis 2,0 Gew.-% beträgt.

18. Zusammensetzung nach Anspruch 1, worin das Konservierungsmittel (b) ein starkes Konservierungsmittel ist und das nichtionische Tensid (e) die Solubilität des Prostagtandinwirkstoffs erhöht, aber die konservierende Wirksamkeit des starken Konservierungsmittels erniedrigt, wobei diese Zusammensetzung weiter noch umfasst
(g) einen Konservierungsmittelverstärker, der die Wirksamkeit des starken Konservierungsmittels erhöht.

19. Zusammensetzung nach Anspruch 18, worin der Konservierungsmittelverstärker aus der Gruppe ausgewählt ist, die besteht aus Mannit, Ethylendiamintetraessigsäure, Salzen hiervon und. Gemischen hiervon.

20. Zusammensetzung nach Anspruch 19, worin den Konservierungsmittelverstärker Mannit ist.

21. Zusammensetzung nach Anspruch 19, worin der Konservierungsmittelverstärker Ethylendiamintetraessigsäure ist.

22. Zusammensetzung nach Anspruch 21, worin der Konservierungsmittelverstärker ein Gemisch aus Ethylendiamintetraessigsäure und Mannit ist.

23. Verwendung einer Zusammensetzung nach einem der vorhergehenden Ansprüche zur Herstellung einer ophthalmischen Zusammensetzung zur Behandlung einer Augenhypertension.

## Revendications

1. Composition comprenant :
(a) un agent actif prostaglandine qui est une ester isopropylique de 13,14-dihydro-15-céto-20-éthyl-PGF₂α, l'un de ses sels, ou un mélange de ceux-ci,
(b) un conservateur ophtalmique ;
(c) un agent d'ajustement de la tonicité non ionique, ledit agent d'ajustement de la tonicité non ionique étant présent en une concentration suffisante pour :
(i) ajuster la tonicité de la composition et
(ii) accroître l'efficacité de conservation ;
(d) un support ophtalmiquement acceptable, et
(e) comprenant en outre 0,1 à 5 pour cent en poids d'un agent tensioactif non ionique choisi dans le groupe constitué par les esters d'acides gras de polyoxyéthylènesorbitan, les éthers alkyliques de polyoxyethylène, et leurs mélanges.

2. Composition selon la revendication 1, comprenant en outre un agent chélatant.

3. Composition selon la revendication 2, dans laquelle ledit agent chélatant est l'acide éthylènediaminetétraacétique on l'un de ses sels.

4. Composition selon la revendication 2, comprenant 0,1 à 10 pour cent en poids d'un agent d'ajustement de la tonicité non ionique et 0,01 à 0,10 pour cent en poids d'un agent chélatant.

5. Composition selon la revendication 1, comprenant 0,001 à 0,30 pour cent en poids d'agent actif.

6. Composition selon la revendication 5, comprenant 0,06 à 0,24 pour cent en poids d'agent actif.

7. Composition selon la revendication 6, comprenant 0,10 à 0,20 pour cent en poids d'agent actif.

8. Composition selon la revendication 1, dans laquelle l'agent tensioactif non ionique est le Polysorbate 80 et est présent en une concentration comprise entre 0,3 et 2,0 pour cent en poids.

9. Composition selon la revendication 1, comprenant 0,001 à 0,05 pour cent en poids d'un conservateur ophtalmique choisi dans le groupe constitué par le sulfate de polymyxine B; les composés d'ammonium quaternaire ; les composés de poly(ammonium quaternaire) ; les esters d'acide p-hydroxybenzoïque ; certains phénols et alcools substitués ; le chlorure de benzalkonium ; le chlorure de benzoxonium ; le chlorure de cétylpyridinium; le chlorure de benzéthonium; le bromure de cétyltriméthylammonium; la chlorhexidine; le poly(hexaméthylènebiguanide); l'acide 1-octanesulfonique (sel monosodique); l'acide 9-octadécénoïque (sulfoné); la ciprofloxacine; le dodécyldiphényloxydeacide disulfonique; les sels d'ammonium, de potassium ou de sodium de l'acide dodécylbenzènesulfonique; les sels de sodium d'acides gras ou de tall oil ; l'acide naphtalenesulfonique ; les sels de sodium de l'acide oléique sulfoné ; les composés mercuriels organiques, tels que le thimérosal ; le thimerfonate-sodium; la 2,3-dichloro-1,4-naphtoquinone; le 3-méthyl-4-chlorophénol ; la 8-hydroxyquinoléine ; l'alcool benzylique; les bis(hydtoxyphényl)alcanes; les bisphénols; le chlorobutanol; le chloroxylénol ; le dichlorophène [2,2'-méthylène-bis-(4-chlorophénol)] ; les dérivés ortho-alkyliques de parabromophénol et de parachlorophénol ; l'oxyquinoléine ; les dérivés para-alkyliques d'orthochlorophénol et d'orthobromophénol; le laurate de pentachlorophényle; les dérivés phénoliques tels que le 2-phénylphénol, le 2-benzyl-4-chlorophénol, le 2-cyclopéntyl-4-chlorophénol, le 4-t-amylphénol, le 4-t-butylphénol, et les 4-et 6-chloro-2-pentylphénol; le polyester d'acide gras phénoxylique; le phénoxyéthanol, l'alcool phényléthylique ; les dérivés et les mélanges de ceux-ci,

10. Composition selon la revendication 1, comprenant en outre un conservateur faible choisi dans le groupe, constitué par des peroxydes, des entités générant des peroxydes, et leurs mélanges.

11. Composition selon la revendication 1, comprenant en outre un tampon ophtalmiquement acceptable, choisi dans le groupe constitué par l'EDTA, les borates et les phosphates, dans laquelle ledit tampon est présent en quantité suffisante pour maintenir la composition à un pH de 4,5 à 8,0 pendant une période pouvant atteindre une année de stockage.

12. Composition selon la revendication 1, comprenant 0,1 à 10 pour cent en poids d'un agent d'ajustement de la tonicité non ionique ophtalmiquement acceptable, choisi dans le groupe constitué par le mannitol, le sorbitol, le glycérol, les polyéthylèneglycols (PEG), les polypropylèneglycols (PPG), et leurs mélanges.

13. Composition selon la revendication 5, dans laquelle le conservateur ophtalmique (b) est présent en une concentration comprise entre 0,005 et 0,2 pour cent en poids.

14. Composition selon la revendication 1, comprenant :
(1) un agent actif prostaglandine ;
(2) un premier agent tensioactif non ionique ; et
(3) un second agent tensioactif non ionique ; et
(4) un support ophtalmiquement acceptable ;
dans laquelle la concentration totale d'agent tensioactif est intérieure à la concentration d'agent tensioactif qui serait nécessaire pour solubiliser l'agent actif prostaglandine pour chaque agent tensioactif non ionique individuel.

15. Composition selon la revendication 14, dans laquelle ledit premier agent tensioactif non ionique est un ester d'acide gras de polyoxyéthylènesorbitan et ledit second agent tensioactif non ionique est un ether alkylique de polyoxyéthylène.

16. Composition selon la revendication 15, dans laquelle ledit premier agent tensioactif non ionique est un composé Polysorbate et ledit second agent tensioactif non ionique est un composé Brij.

17. Composition selon la revendication 16, dans laquelle la concentration totale en agent tensioactif non ionique dans la composition est comprise entre 0,3 et 2,0 pour cent en poids.

18. Composition selon la revendication 1, dans laquelle ledit conservateur (b) est un conservateur fort et ledit agent tensioactif non ionique (e) augmente la solubilité de l'agent actif prostaglandine, mais diminue l'efficacité de conservation du conservateur fort ; ladite composition comprenant en outre :
(g) un amplificateur de conservation qui augmente l'efficacité du conservateur fort.

19. Composition selon la revendication 18, dans laquelle ledit amplificateur de conservation est choisi dans le groupe constitué par le mannitol, l'acide éthylènediaminetétraacétique, leurs sels, et leurs mélanges.

20. Composition selon la revendication 19, dans laquelle ledit amplificateur de conservation est le mannitol.

21. Composition selon la revendication 19, dans laquelle ledit amplificateur de conservation est l'acide éthylènediaminetétraacétique.

22. Composition selon la revendication 21, dans laquelle ledit amplificateur de conservation est un mélange d'acide éthylènediaminetétraacétique et de mannitol.

23. Utilisation d'une composition selon l'une quelconque des revendications précédentes dans la préparation d'une composition ophtalmique pour le traitement de l'hypertension oculaire.
